# EUROPEAN PATENT SPECIFICATION

(11) **EP 1 515 928 B1**
(45) Date of publication and mention of the grant of the patent: **23.05.2007**
(21) Application number: 02708619.8
(22) Date of filing: 21.03.2002
(51) Int. Cl.: C07C 2/86, C07C 2/66

(54) **PROCESS FOR THE PREPARATION OF DIMETHYLCUMENES**
VERFAHREN ZUR HERSTELLUNG VON DIMETHYLCUMOLEN
PROCEDE DE PREPARATION DE DIMETHYLCUMENES

(43) Date of publication of application: 23.03.2005
(73) Proprietor: COUNCIL OF SCIENTIFIC AND INDUSTRIAL RESEARCH, New Delhi 110 001 (IN)
(72) Inventor: PATRA, Chitta Ranjan, National Chemical Laboratory, Maharashtra (IN); KUMAR, Rajiv, National Chemical Laboratory, Maharashtra (IN)
(74) Representative: Degret, Jacques
(86) International application number: PCT/IN2002/000060
(87) International publication number: WO 2003/080547

(56) References cited:
- EP-A- 0 371 738
- EP-A- 0 439 632
- EP-A- 0 538 518
- EP-A- 0 940 178
- EP-A- 1 069 099
- EP-A- 1 069 100
- US-A- 3 553 274

## Description

### Field of the invention

The present invention relates to a process for the preparation of dimethylcumenes. More particularly, the present invention relates to a process for the preparation of dimethylcumenes by the catalytic isopropylation of xylenes using isopropylene as the alkylating agent over a solid catalyst.

### Background of the invention

Dimethylcumenes are useful raw materials for the production of xylenols, pesticides, pharmaceuticals, perfumery, heat transfer media, polymers and special solvents. Xylenols are an important class of phenolic substrates useful as raw materials for making phenolic resins having specific thermoelastic properties.

The most common method for the preparation of dimethylcumenes is by the alkylation of xylene carried out in the presence of metal halides, AlCl₃, ZnCl₂ and inorganic acids HCl, H₂SO₄ [(i) Journal of Chemical Education, Vol. 70 (6), pages A 152 -154, 1993; and (ii) US Patent 5,300,717, which disclose a process for the preparation of dixylylpropane wherein one step comprises the formation of 1,2-dimethyl-4-isopropylbenzene from o-xylene and propene in the presence of conventional Friedel Crafts catalysts such as BF₃ - H₃PO₄, AlCl₃ -CH₃NO₂). This process suffers from the disadvantage that it is environmentally hazardous, giving rise to problems in terms of handling, safety, corrosion and waste disposal.

Rare earth modified zeolites such as (Nd-Na-Y) are reported as catalyzing the alkylation, including the isopropylation of o-xylene (Dokl. Akad. Nauk., Vol. 335 (3), 1994, p. 322 - 325 < CA: 121:182357). In this case, although the isopropylation of o-xylene resulted in the formation of a mixture of dimethylcumenes, alkylation could not be obtained when *p*-xylene was used as a substrate. The disadvantage of this catalyst system is its inactivity for other xylene isomers such *p*-xylene. The conversions and selectivity are also low for the desired dimethylcumene isomers. Additionally, this catalyst is less active for isopropylation as compared to tertiary butylation of o-xylene. Solid catalysts such as TiO₂-SiO₂-Al₂O₃ (Tisial) and MoO₃- SiO₂-Al₂O₃ (Mosial) are also reported for the catalytic alkylation of benzene, toluene and xylene isomers with C₂, C₃, and C₄ aliphatic alcohols (Indian Journal of Chemistry, Vol. 33B, pp. 1053 - 1061, 1994). However, the yield of desired mono dimethylcumenes is low (ca.50%).

In view of the above-mentioned drawbacks and limitations of the above process, it was found desirable to develop an improved process for the production of dimethylcumene isomers with isopropanol using a solid acid catalyst, either in batch reactor or in fixed bed down flow reactor.

### Objects of the invention

The main object of the present invention is to provide an improved process for the preparation of dimethylcumenes overcoming the above-mentioned drawbacks by contacting xylene isomers with isopropanol using a solid acid catalyst with high yield and selectivity.

It is another object of the present invention to sue solid heterogeneous catalyst which are reusable by simple thermal treatment in the presence of air.

Yet another object of the invention is to provide a process for the preparation of dimethylcumenes in either batch reactor or in a continuous fixed bed reactor.

### Summary of the invention

Accordingly, the present invention relates to a process for the preparation of dimethylcumenes comprising alkylating a substrate comprising of one or more xylene isomers with an alkylating agent in the presence of a solid acid zeolite catalyst, and separating the products formed in vapour phase.

In one embodiment of the invention, said substrate and alkylating agent are contacted with said solid acid zeolite catalyst at a temperature in the range of 80 - 250°C and for a period of at least 1 hour.

The product is separated from the vapour phase by condensation at a temperature in the range of 0 - 3°C.

In yet another embodiment of the invention, the substrate is selected from *o*-xylene, *m*-xylene, *p*-xylene and any mixture thereof.

The solid acid zeolite catalyst comprises a solid heterogeneous solid acid catalyst selected from ultrastable zeolite Y (Si/Al = 5 to 50) and Beta (Si/Al= 10 -120), preferably, the Si/Al ratio in said catalyst is between 5 to 20.

The alkylating agent is selected from propylene and propyl alcohols such as isopropanol and n-propanol.

In another embodiment of the invention, the temperature of the reaction is in the range of 100 - 200°C, preferably 120 - 180°C.

In yet another embodiment of the invention, the molar ratio of xylene substrate to the alkylating agent in the feed is in the range of from 1:2 to 20:1, preferably 1:1 to 10:1, more preferably, in the range of 1:2 to 5:1.

In another embodiment of the invention, the weight hourly space velocity (WHSV) of the feed is in the range of 0.5 to 30 h⁻¹, preferably 1 to 20h⁻¹, more preferably 2 to 10h⁻¹.

In a further embodiment of the invention, the alkylation reaction is carried out in a fixed bed reactor or a batch reactor.

In yet another embodiment of the invention, *p*-xylene is alkylated using isopropanol in the presence of zeolite beta catalyst.

In another embodiment of the invention, *m*-xylene and *o*-xylene are alkylated using isopropanol as the alkylating agent in the presence of ultrastable zeolite Y (USY) as catalyst.

In yet another embodiment of the invention, a mixture of *p*-xylene and isopropyl alcohol in a molar ratio of 4:1 is reacted in a fixed bed reactor in the presence of ultrastable zeolite Y (hereinafter USY) catalyst.

### Detailed description of the invention

The present invention provides a process for the preparation of dimethylcumenes by reacting a xylene isomer or a mixture of xylene isomers with an alkylating agent in presence of a solid acid zeolite catalyst. The reaction can be carried out either in a fixed bed reactor or a batch reactor. Preferably, the temperature of the alkylation reaction is in the range of 80 - 250°C, and the molar ratio of the xylene isomer substrate to the alkylating agent in the feed is in the range of 1:2 to 20:1. Reaction is carried out at a WHSV of 0.5 to 30h⁻¹. Propylene or propyl alcohols such as isopropanol or n-propanol are used as alkylating agents.

The novelty and inventive step in this invention resides in the use of solid acid zeolite catalysts such as zeolite USY or zeolite beta as the catalysts for alkylation reaction. Solid acid zeolite catalysts show very high catalytic activity and selectivity in alkylation of all xylene isomers, unlike in the prior art wherein only *o*-xylene and not m- or p-xylenes could be alkylated using Nd-Na-Y zeolite catalyst. While both zeolite USY and zeoltie Beta are suitable for propylation of xylene isomers, it is observed that zeolite Beta is more suitable for isopropylation of *p*-xylene and zeolite USY is preferred for isopropylation of *m-* and *p-*xylene. Without wishing to be bound by any theory, it is believed that this could be due to the slightly small pore openings of zeolite Beta as compared to zeolite USY. *p*-xylene being the smallest among all the xylenes, has faster ingress into zeolite Beta when compared to ortho- or meta- isomers. This diffusional limitation is not present in the case of zeolite USY. H-Mord. and MFI are not as good catalysts as zeolite Beta or zeolite USY for the alkylation reaction of this invention, due to their smaller pore size. *p*-xylene yields only one dimethylcumene isomer, 2,5 dimethylcumene since all regio positions are the same. *o*-xylene yields 2,3 dimethylecumene and 3,4 dimethylcumene isomers as the primary products. While in principle *m*-xylene can yield three dimethylcumene isomers, 2,4 dimethylcumene, 2,6 dimethylcumene and 3,5 dimethylcumene, only two isomers, 2,4 dimethylcumene, 2,6 dimethylcumene are formed as primary products since the alkylation is an ortho-para directing electrophilic substitution reaction and the regio position 3,5 in *m*-xylene is deactivated due to the meta position from both the methyl groups in *m*-xylene.

The following examples are provided to illustrate the invention and should not be construed as limiting the scope of the invention.

### Example 1

This example illustrates effect of time on stream (TOS) on conversion and product selectivity in isopropylation of *p*-xylene. USY catalyst (0.5g) was loaded into the reactor such that the catalyst bed was sandwiched between inert porcelain beads. A mixture of *p*-xylene and isopropyl alcohol in a 4:1 molar ratio was introduced into fixed bed reactor by syringe pump (Sage Instruments, Model 352, USA) in a continuous manner in presence of inert carrier gas (nitrogen, flow = 35 ml/min) at a WHSV of 6.48 h⁻¹ and a temperature of 140°C for a period of 8 hours. Product samples were collected periodically (Table I below), chilled at 0°C and analyzed by gas chromatograph (Shimadzu GC - 14B) using flame ionization detector and 3m x 1/8" packed column with 5% bentone and 5% DIDP on chromosorb WHP, with mesh size of 801100. Results of the reaction are given in Table I below.

**Table I: Effect of TOS on conversion and product selectivity in the isopropylation of p-xylene over zeolite USY**

| Conversion or selectivity (mole %) | Time on stream | | | |
|---|---|---|---|---|
| | 1h | 3h | 5h | 8h |
| Conversion of *p*-xylene | 16.2 | 16.5 | 14.6 | 10.0 |
| Selectivity of dimethylcumenes in total products^{a} | 94.2 | 88.8 | 87.8 | 84.0 |
| Selectivity of 2,5 dimethylcumene among DMC's | 100 | 100 | 100 | 100 |
| Selectivity of other products in total products | 5.8 | 11.2 | 12.2 | 16.0 |
| % yield of DMC in total products w.r.t. limiting reagent, i.e. isopropylating agent | 61.2 | 58.8 | 51.2 | 33.6 |

| | | | | |
|---|---|---|---|---|
| ^{a} the remaining products were mainly other isomerised and disproportionated products of xylene along with diisopropyl xylene (DIPX). | | | | |

### Example 2

This example illustrates effect of temperature on conversion and product selectivity in isopropylation of *p*-xylene. USY catalyst (0.5g) was loaded into reactor such that the catalyst bed was sandwiched between inert porcelain beads. A mixture of *p*-xylene and isopropyl alcohol in 4:1 molar ratio was introduced into fixed bed reactor by syringe pump (Sage Instruments, Model 352, USA) in continuous manner in presence of inert carrier gas (nitrogen, flow = 35 ml/min) at WHSV of 6.48 h⁻¹ and at different temperatures (Table II) for a period of 1 hour. Products were chilled at 0°C and analyzed by gas chromatograph using flame ionization detector. Results of reaction are given in Table II.

**Table II: Effect of temperature on conversion and product selectivity in the isopropylation of p-xylene over zeolite USY**

| Conversion or selectivity (mole %) | Temperature, °C | | | |
|---|---|---|---|---|
| | 120 | 140 | 160 | 180 |
| Conversion of *p*-xylene | 6.9 | 16.2 | 22.6 | 28.0 |
| Selectivity of dimethylcumenes in total products^{a} | 96.5 | 94.2 | 78.6 | 56.1 |
| Selectivity of 2,5 dimethylcumene among DMC's | 100 | 100 | 97.0 | 89.0 |
| Selectivity of other products in total products | 3.5 | 5.8 | 21.4 | 43.9 |
| % yield of DMC in total products w.r.t. limiting reagent, i.e. isopropylating agent | 26.8 | 61.2 | 71.2 | 62.8 |

| | | | | |
|---|---|---|---|---|
| ^{a} the remaining products were mainly other isomerised and disproportionated products of xylene along with diisopropyl xylene (DIPX). | | | | |

### Example 3

USY catalyst (0.5g) was loaded into the reactor such that the catalyst bed was sandwiched between inert porcelain beads. A mixture of *p*-xylene and isopropyl alcohol in a 4:1 molar ratio was introduced into fixed bed reactor by syringe pump (Sage Instruments, Model 352, USA) in a continuous manner in the presence of inert carrier gas (nitrogen, flow = 35 ml/min) at different WHSV (See Table III) and a temperature of 140°C for 1 hour. Product was chilled at 0°C and analyzed by gas chromatograph using flame ionization detector. Results of reaction are given in Table III.

**Table III: Effect of space velocity on conversion and product selectivity in the isopropylation of p-xylene over zeolite USY**

| Conversion or selectivity (mole %) | WHSV, h⁻¹ | | |
|---|---|---|---|
| | 3.2 | 6.5 | 12.9 |
| Conversion of *p*-xylene | 24.3 | 16.2 | 11.8 |
| Selectivity of dimethylcumenes in total products^{a} | 90.5 | 94.2 | 96.2 |
| Selectivity of 2,5 dimethylcumene among DMC's | 98 | 100 | 100 |
| Selectivity of other products in total products | 9.5 | 5.8 | 3.8 |
| % yield of DMC in total products w.r.t. limiting reagent, i.e. isopropylating agent | 88.0 | 61.2 | 45.6 |

| | | | |
|---|---|---|---|
| ^{a} the remaining products were mainly other isomerised and disproportionated products of xylene along with diisopropyl xylene (DIPX). | | | |

### Example 4

This example illustrates effect of molar ratio of *p*-xylene to isopropanol on conversion and product selectivity in isopropylation of *p*-xylene. USY catalyst (0.5g) was loaded into the reactor such that catalyst bed was sandwiched between inert porcelain beads. A mixture of *p-*xylene and isopropyl alcohol with different molar ratio (Table IV) was introduced into fixed bed reactor by syringe pump (Sage Instruments, Model 352, USA) in a continuous manner in the presence of inert carrier gas (nitrogen, flow = 35 ml/min) at a WHSV of 6.48 h⁻¹ and a temperature of 140°C for a period of 8 hours. Products were chilled at 0°C and analyzed by gas chromatograph using flame ionization detector. Results of reaction are given in Table IV.

**Table IV: Effect of molar ratio of p-xylene and isopropanol on conversion and product selectivity in the isopropylation of p-xylene over zeolite USY**

| Conversion or selectivity (mole %) | *p*-xylene /isopropanol (molar) | | | |
|---|---|---|---|---|
| | 2:1 | 4:1 | 8:1 | 10:1 |
| Theoretical maximum conversion | 50 | 25 | 12.5 | 10 |
| Conversion of *p*-xylene | 35.4 | 16.2 | 9.7 | 7.1 |
| Selectivity of dimethylcumenes in total products^{a} | 76.8 | 94.2 | 95.6 | 100 |
| Selectivity of 2,5 dimethylcumene among DMC's | 97.3 | 100 | 100 | 100 |
| Selectivity of other products in total products | 23.2 | 5.8 | 4.4 | 0 |
| % yield of DMC in total products w.r.t. limiting reagent, i.e. isopropylating agent | 54.4 | 61.2 | 74.4 | 71.0 |

| | | | | |
|---|---|---|---|---|
| ^{a} the remaining products were mainly other isomerised and disproportionated products of xylene along with diisopropyl xylene (DIPX). | | | | |

### Example 5

This example illustrates effect of time on stream (TOS) on conversion and product selectivity in isopropylation of *m*-xylene. USY catalyst (0.5g) was loaded into a reactor such that the catalyst bed was sandwiched between inert porcelain beads. A mixture of *m*-xylene and isopropyl alcohol in a 4:1 molar ratio was introduced into fixed bed reactor by a syringe pump (Sage Instruments, Model 352, USA) in a continuous manner in presence of inert carrier gas (nitrogen, flow = 35 ml/min) at a WHSV of 6.48 h⁻¹ and a temperature of 140°C for a period of 1-8 hours. Products were chilled at 0°C, collected every hour up to 8 hours and analyzed by gas chromatograph (Shimadzu GC - 14B) using flame ionization detector and 3m x 1/8" packed column with 5% bentone and 5% DIDP on chromosorb WHP, with mesh size of 801100. Results of the reaction are given in Table V.

**Table V: Effect of TOS on conversion and product selectivity in the isopropylation of m-xylene over zeolite USY**

| Conversion or selectivity (mole %) | Time on stream | | | |
|---|---|---|---|---|
| | 1h | 3h | 5h | 8h |
| Conversion of *m*-xylene | 16.6 | 19.1 | 15.7 | 14.2 |
| Selectivity of dimethylcumenes in total products^{a} | 91.0 | 93.0 | 91.7 | 87.0 |
| Selectivity of 2,4 dimethylcumene among DMC's | 78.9 | 56.1 | 52.8 | 29.6 |
| Selectivity of 2,6 dimethylcumene among DMC's | 21.1 | 43.9 | 47.2 | 70.4 |
| Selectivity of other products in total products | 9.0 | 7.0 | 8.3 | 13.0 |
| 2.4 DMC/2.6 DMC | 3.7 | 1.3 | 1.1 | 0.4 |
| % yield of DMC in total products w.r.t. limiting reagent, i.e. isopropylating agent | 60.0 | 70.8 | 57.6 | 49.6 |

| | | | | |
|---|---|---|---|---|
| ^{a} the remaining products were mainly other isomerised and disproportionated products of xylene along with diisopropyl xylene (DIPX). | | | | |

### Example 6

This example illustrates effect of temperature on conversion and product selectivity in isopropylation of *m*-xylene. USY catalyst (0.5g) was loaded into reactor such that the catalyst bed was sandwiched between inert porcelain beads. A mixture of *m*-xylene and isopropyl alcohol in 4:1 molar ratio was introduced into fixed bed reactor by a syringe pump (Sage Instruments, Model 352, USA) in continuous manner in presence of an inert carrier gas (nitrogen, flow = 35 ml/min) at WHSV of 6.48 h⁻¹ and at different temperatures (Table VI) for a period of 1 hour. Products were chilled at 0°C and analyzed by gas chromatograph using flame ionization detector. Results of reaction are given in Table VI.

**Table VI: Effect of temperature on conversion and product selectivity in the isopropylation of m-xylene over zeolite USY**

| Conversion or selectivity (mole %) | Temperature, °C | | | |
|---|---|---|---|---|
| | 120 | 140 | 160 | 180 |
| Conversion of *m*-xylene | 12.3 | 16.5 | 27.3 | 29.3 |
| Selectivity of dimethylcumenes in total products^{a} | 93.3 | 91.0 | 88.0 | 64.1 |
| Selectivity of 2,4 dimethylcumene among DMC's | 58.8 | 78.9 | 75.3 | 67.2 |
| Selectivity of 2,6 dimethylcumene among DMC's | 41.2 | 21.1 | 24.7 | 32.8 |
| Selectivity of other products in total products | 6.7 | 9.0 | 12.0 | 35.9 |
| 2,4 DMC/2,6 DMC | 1.4 | 3.7 | 3.1 | 2.0 |
| % yield of DMC in total products w.r.t. limiting reagent, i.e. isopropylating agent | 46.0 | 60.0 | 96.1 | 75.2 |

| | | | | |
|---|---|---|---|---|
| ^{a} the remaining products were mainly other isomerised and disproportionated products of xylene along with diisopropyl xylene (DIPX). | | | | |

### Example 7

USY catalyst (0.5g) was loaded into the reactor such that the catalyst bed was sandwiched between inert porcelain beads. A mixture of *m*-xylene and isopropyl alcohol in a 4:1 molar ratio was introduced into the fixed bed reactor by a syringe pump (Sage Instruments, Model 352, USA) in a continuous manner in the presence of an inert carrier gas (nitrogen, flow = 35 ml/min) at different WHSV (Table VII) and a temperature of 140°C for 1 hour.

Product was chilled at 0°C and analyzed by gas chromatograph using flame ionization detector. Results of the reaction are given in Table VII below.

**Table VII: Effect of space velocity on conversion and product selectivity in the isopropylation of m-xylene over zeolite USY**

| Conversion or selectivity (mole %) | WHSV, h⁻¹ | | |
|---|---|---|---|
| | 3.2 | 6.5 | 12.9 |
| Conversion of *m*-xylene | 20.7 | 16.5 | 10.7 |
| Selectivity of dimethylcumenes in total products^{a} | 90.0 | 91.0 | 93.8 |
| Selectivity of 2,4 dimethylcumene among DMC's | 75.8 | 78.9 | 53.3 |
| Selectivity of 2,6 dimethylcumene among DMC's | 24.2 | 21.1 | 46.5 |
| Selectivity of other products in total products | 10.0 | 9.0 | 6.2 |
| 2,4 DMC/2,6 DMC | 3.1 | 3.7 | 1.2 |
| % yield of DMC in total products w.r.t. limiting reagent, i.e. isopropylating agent | 74.4 | 60.0 | 40.0 |

| | | | |
|---|---|---|---|
| ^{a} the remaining products were mainly other isomerised and disproportionated products of xylene along with diisopropyl xylene (DIPX). | | | |

### Example 8

This example illustrates the effect of molar ratio of *m*-xylene to isopropanol on conversion and product selectivity in isopropylation of *m*-xylene. USY catalyst (0.5g) was loaded into the reactor in such a way that the catalyst bed was sandwiched between inert porcelain beads. A mixture of *m*-xylene and isopropyl alcohol with different molar ratio (Table VIII) was introduced into fixed bed reactor by a syringe pump (Sage Instruments, Model 352, USA) in a continuous manner in the presence of an inert carrier gas (nitrogen, flow = 35 ml/min) at a WHSV of 6.48 h⁻¹ and a temperature of 140°C for a period of 8 hours. The product were chilled at 0°C and analyzed by gas chromatograph using flame ionization detector. Results of the reaction are given in Table VIII.

**Table VIII: Effect of molar ratio of m-xylene and isopropanol on conversion and product selectivity in the isopropylation of m-xylene over zeolite USY**

| Conversion or selectivity (mole %) | *m*-xylene /isopropanol (molar) | | | |
|---|---|---|---|---|
| | 2:1 | 4:1 | 8:1 | 10:1 |
| Theoretical maximum conversion | 50 | 25 | 12.5 | 10 |
| Conversion of *m*-xylene | 19.1 | 16.5 | 11.3 | 6.7 |
| Selectivity of dimethylcumenes in total products^{a} | 74.6 | 91.0 | 98.0 | 99.0 |
| Selectivity of 2,4 dimethylcumene among DMC's | 54.2 | 78.9 | 71.1 | 72.0 |
| Selectivity of 2,6 dimethylcumene among DMC's | 45.8 | 21.1 | 28.9 | 28.0 |
| Selectivity of other products in total products | 25.4 | 9.0 | 2.0 | 1.0 |
| 2,4 DMC/2,6DMC | 1.2 | 3.7 | 2.5 | 2.6 |
| % yield of DMC in total products w.r.t. limiting reagent, i.e. isopropylating agent | 28.6 | 60.0 | 88.8 | 66.0 |

| | | | | |
|---|---|---|---|---|
| ^{a} the remaining products were mainly other isomerised and disproportionated products of xylene along with diisopropyl xylene (DIPX). | | | | |

### Example 9

This example illustrates effect of time on stream (TOS) on conversion and product selectivity in isopropylation of o-xylene. USY catalyst (0.5g) was loaded into the reactor such that the catalyst bed was sandwiched between inert porcelain beads. A mixture of o-xylene and isopropyl alcohol in a 4:1 molar ratio was introduced into fixed bed reactor by a syringe pump (Sage Instruments, Model 352, USA) in a continuous manner in the presence of inert carrier gas (nitrogen, flow = 35 ml/min) at a WHSV of 6.48 h⁻¹ and a temperature of 140°C for a period of 1-8 hours. Products were chilled at 0°C, collected every hour up to 8 hours and analyzed by gas chromatograph (Shimadzu GC - 14B) using flame ionization detector and 3m x 1/8" packed column with 5% bentone and 5% DIDP on chromosorb WHP, with mesh size of 801100. Results of reaction are given in Table IX.

**Table IX: Effect of TOS on conversion and product selectivity in the isopropylation of o-xylene over zeolite USY**

| Conversion or selectivity (mole %) | Time on stream | | | |
|---|---|---|---|---|
| | 1h | 3h | 5h | 8h |
| Conversion of *o*-xylene | 15.6 | 18.6 | 16.9 | 15.1 |
| Selectivity of dimethylcumenes in total products^{a} | 85.1 | 85.0 | 83.8 | 80.0 |
| Selectivity of 3,4 dimethylcumene among DMC's | 92.6 | 90.9 | 89.7 | 85.4 |
| Selectivity of 2,3 dimethylcumene among DMC's | 6.4 | 8.4 | 9.7 | 14.1 |
| Selectivity of other products in total products | 14.9 | 15.0 | 16.2 | 20.0 |
| 3,4 DMC/2,3 DMC | 14.5 | 10.8 | 9.2 | 6.1 |
| % yield of DMC in total products w.r.t. limiting reagent, i.e. isopropylating agent | 53.2 | 63.2 | 56.8 | 48.4 |

| | | | | |
|---|---|---|---|---|
| ^{a} the remaining products were mainly other isomerised and disproportionated products of xylene along with diisopropyl xylene (DIPX). | | | | |

### Example 10

This example illustrates effect of temperature on conversion and product selectivity in isopropylation of *o*-xylene. USY catalyst (0.5g) was loaded into reactor such that the catalyst bed was sandwiched between inert porcelain beads. A mixture of *o*-xylene and isopropyl alcohol in 4:1 molar ratio was introduced into fixed bed reactor by a syringe pump (Sage Instruments, Model 352, USA) in continuous manner in presence of inert carrier gas (nitrogen, flow = 35 ml/min) at WHSV of 6.48 h⁻¹ and at different temperatures (Table X) for a period of 1 hour. Products were chilled at 0°C and analyzed by gas chromatograph using flame ionization detector. Results of reaction are given in Table X.

**Table X: Effect of temperature on conversion and product selectivity in the isopropylation of o-xylene over zeolite USY**

| Conversion or selectivity (mole %) | Temperature, °C | | | |
|---|---|---|---|---|
| | 120 | 140 | 160 | 180 |
| Conversion of *o*-xylene | 4.6 | 15.6 | 20.7 | 25.0 |
| Selectivity of dimethylcumenes in total products^{a} | 86.0 | 85.1 | 83.0 | 75.7 |
| Selectivity of 3,4 dimethylcumene among DMC's | 72.3 | 92.6 | 95.0 | 95.6 |
| Selectivity of 2,3 dimethylcumene among DMC's | 26.5 | 6.4 | 2.9 | 2.6 |
| Selectivity of other products in total products | 14.0 | 14.9 | 17.0 | 24.3 |
| 3,4 DMC/2,3 DMC | 2.7 | 14.5 | 32.8 | 36.8 |
| % yield of DMC in total products w.r.t. limiting reagent, i.e. isopropylating agent | 16.0 | 53.2 | 68.8 | 75.6 |

| | | | | |
|---|---|---|---|---|
| ^{a} the remaining products were mainly other isomerised and disproportionated products of xylene along with diisopropyl xylene (DIPX). | | | | |

### Example 11

USY catalyst (0.5g) was loaded into the reactor such that the catalyst bed was sandwiched between inert porcelain beads. A mixture of o-xylene and isopropyl alcohol in a 4:1 molar ratio was introduced into fixed bed reactor by a syringe pump (Sage Instruments, Model 352, USA) in a continuous manner in the presence of inert carrier gas (nitrogen, flow = 35 ml/min) at different WHSV (Table XI) and a temperature of 140°C for 1 hour. Product was chilled at 0°C and analyzed by gas chromatograph using flame ionization detector. Results of reaction are given in Table XI.

**Table XI: Effect of space velocity on conversion and product selectivity in the isopropylation of o-xylene over zeolite USY**

| Conversion or selectivity (mole %) | WHSV, h⁻¹ | | |
|---|---|---|---|
| | 3.2 | 6.5 | 12.9 |
| Conversion of *o*-xylene | 24.3 | 15.6 | 6.4 |
| Selectivity of dimethylcumenes in total products^{a} | 82.0 | 85.1 | 86.5 |
| Selectivity of 3,4 dimethylcumene among DMC's | 94.6 | 92.6 | 81.3 |
| Selectivity of 2,3 dimethylcumene among DMC's | 4.2 | 6.4 | 18.0 |
| Selectivity of other products in total products | 18.0 | 14.9 | 13.5 |
| 3,4 DMC/2,3 DMC | 22.5 | 14.5 | 4.5 |
| % yield of DMC in total products w.r.t. limiting reagent, i.e. isopropylating agent | 79.6 | 53.2 | 22.0 |

| | | | |
|---|---|---|---|
| ^{a} the remaining products were mainly other isomerised and disproportionated products of xylene along with diisopropyl xylene (DIPX). | | | |

### Example 12

This example illustrates the effect of molar ratio of *o*-xylene to isopropanol on conversion and product selectivity in isopropylation of *o*-xylene. USY catalyst (0.5g) was loaded into the reactor in such a way that the catalyst bed was sandwiched between inert porcelain beads. A mixture of o-xylene and isopropyl alcohol with different molar ratio (See Table XII) was introduced into the fixed bed reactor by a syringe pump (Sage Instruments, Model 352, USA) in a continuous manner in the presence of an inert carrier gas (nitrogen, flow = 35 ml/min) at a WHSV of 6.48 h⁻¹ and a temperature of 140°C for a period of 8 hours.

The product were chilled at 0°C and analyzed by gas chromatograph using flame ionization detector. Results of the reaction are given in Table XII below.

**Table XIII: Effect of molar ratio of o-xylene and isopropanol on conversion and product selectivity in the isopropylation of o-xylene over zeolite USY**

| Conversion or selectivity (mole %) | *o*-xylene/isopropanol (molar) | | | |
|---|---|---|---|---|
| | 2:1 | 4:1 | 8:1 | 10:1 |
| Theoretical maximum conversion | 50 | 25 | 12.5 | 10 |
| Conversion of *o*-xylene | 21.4 | 15.6 | 10.7 | 9.4 |
| Selectivity of dimethylcumenes in total products^{a} | 82.0 | 85.1 | 86.0 | 89.3 |
| Selectivity of 3,4 dimethylcumene among DMC's | 86.5 | 92.6 | 93.4 | 94.6 |
| Selectivity of 2,3 dimethylcumene among DMC's | 13.5 | 6.4 | 6.6 | 5.4 |
| Selectivity of other products in total products | 18.0 | 14.9 | 14.0 | 10.7 |
| 3,4 DMC/2,3 DMC | 6.4 | 14.5 | 14.2 | 17.5 |
| % yield of DMC in total products w.r.t. limiting reagent, i.e. isopropylating agent | 35.0 | 53.2 | 73.6 | 84.0 |

| | | | | |
|---|---|---|---|---|
| ^{a} the remaining products were mainly other isomerised and disproportionated products of xylene along with diisopropyl xylene (DIPX). | | | | |

### Example 13

This example illustrates effect of zeolite structure on conversion of *p*-xylene and the yield of dimethylcumene in the total products. Different catalysts (0.5g) (Table XIII) were loaded into the reactor such that respective catalyst beds were sandwiched between inert porcelain beads. A mixture of *p*-xylene and isopropyl alcohol with molar ratio 4:1 was introduced into the fixed bed reactor by a syringe pump (Sage Instruments, Model 352, USA) in continuous manner in presence of inert carrier gas (nitrogen, flow = 35 ml/min) at WHSV of 3.24 h⁻¹ and temperature of 140°C for 1 hour.

The products were chilled at 0°C and analyzed by gas chromatograph using flame ionization detector. Results of the reaction are given in Table XIII below.

**Table XIII: Effect of different catalysts on conversion and product selectivity in the isopropylation of p-xylene**

| Conversion or selectivity (mole %) | Catalysts | | | |
|---|---|---|---|---|
| | USY | H-Beta | H-Mord | H-ZSM-5 |
| Conversion of *p*-xylene | 24.3 | 21.9 | 11.2 | 0.2 |
| Selectivity of dimethylcumenes in total products^{a} | 90.5 | 97.7 | 97.0 | - |
| Selectivity of 2,5 dimethylcumene among DMC's | 98.0 | 94.4 | 95.6 | - |
| Selectivity of other products in total products | 9.5 | 2.3 | 3.0 | - |
| % yield of DMC in total products w.r.t. limiting reagent, i.e. isopropylating agent | 88.0 | 85.6 | 10.9 | - |

| | | | | |
|---|---|---|---|---|
| ^{a} the remaining products were mainly other isomerised and disproportionated products of xylene along with diisopropyl xylene (DIPX). | | | | |

### Example 14

This example illustrates effect of zeolite structure on conversion of *m*-xylene and the yield of dimethylcumenes. Different catalysts (0.5g) (Table XIV) were loaded into reactor such that the respective catalyst beds were sandwiched between inert porcelain beads. A mixture of *m*-xylene and isopropyl alcohol with molar ratio 4:1 was introduced into the fixed bed reactors by a syringe pump (Sage Instruments, Model 352, USA) in continuous manner in presence of an inert carrier gas (nitrogen, flow = 35 ml/min) at WHSV of 3.24 h⁻¹ and temperature of 140°C for 1 hour.

The products were chilled at 0°C and analyzed by gas chromatograph using flame ionization detector. Results of the reaction are given in Table XIV.

**Table XIV: Effect of different catalysts on conversion and product selectivity in the isopropylation of m-zylene**

| Conversion or selectivity (mole %) | Catalysts | | | |
|---|---|---|---|---|
| | USY | H-Beta | H-Mord | H-ZSM - 5 |
| Conversion of *m*-xylene | 20.7 | 16.5 | 6.0 | 0.1 |
| Selectivity of dimethylcumenes in total products^{a} | 90.0 | 97.5 | 91.4 | - |
| Selectivity of 2,4 dimethylcumene among DMC's | 75.8 | 44.1 | 6.5 | - |
| Selectivity of 2,6 dimethylcumene among DMC's | 24.2 | 55.1 | 93.5 | - |
| Selectivity of other products in total products | 10.0 | 2.5 | 8.6 | - |
| 2,4 DMC/2,6 DMC | 3.1 | 0.8 | 0.07 | - |
| % yield of DMC in total products w.r.t. limiting reagent, i.e. isopropylating agent | 74.4 | 64.4 | 54.8 | - |

| | | | | |
|---|---|---|---|---|
| ^{a} the remaining products were mainly other isomerised and disproportionated products of xylene along with diisopropyl xylene (DIPX). | | | | |

### Example 15

This example illustrates effect of zeolite structure on conversion of *o*-xylene and the yield of dimethylcumenes. Different catalysts (0.5g) (Table XV) were loaded into reactor such that the respective catalyst beds were sandwiched between inert porcelain beads. A mixture of o-xylene and isopropyl alcohol with molar ratio 4:1 was introduced into the fixed bed reactors by a syringe pump (Sage Instruments, Model 352, USA) in continuous manner in presence of an inert carrier gas (nitrogen, flow = 35 ml/min) at WHSV of 3.24 h⁻¹ and temperature of 140°C for 1 hour.

The products were chilled at 0°C and analyzed by gas chromatograph using flame ionization detector. Results of the reaction are given in Table XV.

**Table XV: Effect of different catalysts on conversion and product selectivity in the isopropylation of o-xylene**

| Conversion or selectivity (mole %) | Catalysts | | | |
|---|---|---|---|---|
| | USY | H-Beta | H-Mord | H-ZSM - 5 |
| Conversion of *o*-xylene | 24.2 | 15.5 | 7.5 | 0.0 |
| Selectivity of dimethylcumenes in total products^{a} | 82.0 | 95.9 | 90.5 | - |
| Selectivity of 3,4 dimethylcumene among DMC's | 94.6 | 88.5 | 97.2 | - |
| Selectivity of 2,3 dimethylcumene among DMC's | 4.2 | 9.7 | 2.8 | - |
| Selectivity of other products in total products | 18.0 | 4.1 | 9.5 | - |
| 3,4 DMC/2,3 DMC | 22.5 | 9.1 | 34.7 | - |
| % yield of DMC in total products w.r.t. limiting reagent, i.e. isopropylating agent | 79.2 | 59.6 | 27.2 | - |

| | | | | |
|---|---|---|---|---|
| ^{a} the remaining products were mainly other isomerised and disproportionated products of xylene along with diisopropyl xylene (DIPX). | | | | |

### Example 16

This example illustrates use of batch reactor on conversion and product selectivity in isopropylation of *p*-xylene under autogenous pressure in presence of cyclohexane as solvent using H-Beta catalyst. The reaction was carried out in 100 ml Teflon lined autoclave. A mixture of *p*-xylene (80 mmol) and isopropyl alcohol (20 mmol) in molar ratio 4:1 was added to 50 g cyclohexane in a beaker. The mixture was then transferred to an autoclave and 0.5 g H-Beta (previously activated at 300°C in presence of air) was added to the reaction mixture. The autoclave was then sealed and heated for 25 hours at 190°C oven under self generated pressure. After given reaction time (Table XVI) the autoclave was allowed to cool to room temperature (ca. 2 hours). The solid was extracted with acetone and the mother liquor concentrated in rotavapour.

The concentrated product was analyzed by gas chromatograph (Shimadzu GC -14 B) using flame ionization detector and 3m x 1/8" packed column with 5% bentone and 5% DIDP on chromosorb WHP, with mesh size of 801100. Results of reaction are given in Table XVI.

**Table XVI: Effect of time on stream on conversion and product selectivity in the isopropylation of p-xylene using zeolite H-Beta in batch reactor**

| Conversion or selectivity (mole %) | Reaction time, hours | | | | |
|---|---|---|---|---|---|
| | 3 | 7 | 10 | 20 | 25 |
| Conversion of *p*-xylene | 7.3 | 9.3 | 9.9 | 10.5 | 10.6 |
| Selectivity of dimethylcumenes in total products^{a} | 100 | 100 | 100 | 100 | 100 |
| Selectivity of 2,5 dimethylcumene among DMC's | 100 | 100 | 100 | 100 | 100 |
| Selectivity of other products in total products | 0.0 | 0.0 | 0.0 | 0.0 | 0.0 |
| % yield of DMC in total products w.r.t. limiting reagent, i.e. isopropylating agent | 29.2 | 37.2 | 39.6 | 42.0 | 42.4 |

| | | | | | |
|---|---|---|---|---|---|
| ^{a} the remaining products were mainly other isomerised and disproportionated products of xylene along with diisopropyl xylene (DIPX). | | | | | |

### Example 17

This example illustrates effect of time on stream (TOS) on conversion and product selectivity in alkylation of *p*-xylene using n-propanol as alkylating agent. USY catalyst (0.5g) was loaded into the reactor such that the catalyst bed was sandwiched between inert porcelain beads. A mixture of *p*-xylene and n-propanol in a 4:1 molar ratio (Table XVII) was introduced into fixed bed reactor by syringe pump (Sage Instruments, Model 352, USA) in a continuous manner in presence of inert carrier gas (nitrogen, flow = 35 ml/min) at a WHSV of 3.24 h⁻¹ and a temperature of 140°C for 1 hour. Product were chilled at 0°C, collected and analyzed by gas chromatograph using flame ionization detector. Results of the reaction are given in Table XVII below.

**Table XVII: Effect of TOS on conversion and product selectivity in the isopropylation of p-xylene over zeolite USY using n-propanol as alkylating agent**

| Conversion or selectivity (mole %) | Time on stream | | | |
|---|---|---|---|---|
| | 1h | 3h | 5h | 8h |
| Conversion of *p*-xylene | 6.5 | 5.7 | 4.8 | 3.4 |
| Selectivity of dimethylcumenes in total products^{a} | 88.6 | 90.6 | 96.0 | 98.8 |
| Selectivity of 2,5 dimethylcumene among DMC's | 80.5 | 61.0 | 62.0 | 63.0 |
| Selectivity of other products in total products | 11.4 | 9.4 | 4.0 | 1.2 |
| % yield of DMC in total products w.r.t. limiting reagent, i.e. isopropylating agent | 23.0 | 20.7 | 18.4 | 3.4 |

| | | | | |
|---|---|---|---|---|
| ^{a} the remaining products were mainly other isomerised and disproportionated products of xylene along with diisopropyl xylene (DIPX). | | | | |

### Example 18

This example illustrates effect of time on stream (TOS) on conversion and product selectivity in alkylation of *p*-xylene using propylene as alkylating agent. USY catalyst (0.5g) was loaded into the reactor such that the catalyst bed was sandwiched between inert porcelain beads. A mixture of *p*-xylene and propylene in a 4:1 molar ratio (Table XVIII) was introduced into fixed bed reactor by syringe pump (Sage Instruments, Model 352, USA) in a continuous manner in presence of inert carrier gas (nitrogen, flow = 35 ml/min) at a WHSV of 6.48 h⁻¹ and a temperature of 140°C for a period of 8 hours. Product were chilled at 0°C, collected and analyzed by gas chromatograph using flame ionization detector. Results of the reaction are given in Table XVIII.

**Table XVIII: Effect of TOS on conversion and product selectivity in the isopropylation of p-xylene over zeolite USY using propylene as alkylating agent**

| Conversion or selectivity (mole %) | Time on stream | | | |
|---|---|---|---|---|
| | 1h | 3h | 5h | 8h |
| Conversion of *p*-xylene | 22.3 | 17.2 | 13.8 | 12.5 |
| Selectivity of dimethylcumenes in total products^{a} | 95.4 | 91.8 | 89.6 | 86.4 |
| Selectivity of 2,5 dimethylcumene among DMC's | 98.5 | 98.0 | 99.6 | 100 |
| Selectivity of other products in total products | 4.6 | 8.2 | 10.4 | 13.6 |
| % yield of DMC in total products w.r.t. limiting reagent, i.e. isopropylating agent | 70.0 | 63.2 | 49.6 | 43.2 |

| | | | | |
|---|---|---|---|---|
| ^{a} the remaining products were mainly other isomerised and disproportionated products of xylene along with diisopropyl xylene (DIPX). | | | | |

The salient features of the invention are that the reaction is carried out in a single step by direct contacting a mixture of xylene isomers and alkylating agent in continuous or batch processes with a solid acid zeolite catalyst (preferably USY or Beta). It is observed that the solid acid zeolite catalyst used show high activity and selectivity in the alkylation of all xylene isomers, unlike in the prior art where only o- and not p- or *m*-xylene could be alkylated.

### Advantages of the invention

1. The catalyst used is eco-friendly, easy to handle, easy to recover and has no or minimal corrosion. As a result disposal of waste is not a problem
2. The catalysts exhibit high activity and selectivity in alkylation of all xylene isomers and are easily regenerated by thermal treatment in the presence of air.
3. Dimethylcumene isomers are formed with high selectivity.

## Claims

1. A process for the preparation of dimethylcumenes in vapour phase, the process comprising alkylating a substrate comprising of one or more xylene isomers with an alkylating agent selected from the group consisting of propylene, isopropanol and n-propanol, in the presence of a solid acid zeolite catalyst selected from ultrastable zeolite Y having a Si/Al ratio in the range of 5 to 50 and a Zeolite Beta having a Si/Al ratio in the range of 10 to 120, and separating the products formed in vapour phase by condensation at a temperature in the range of 0-3°C.

2. A process as claimed in claim 1 wherein said substrate and alkylating agent are contacted with said solid acid zeolite catalyst at a temperature in the range of 80 - 250°C and for a period of at least 1 hour.

3. A process as claimed in claim 1 wherein the substrate is selected from *o*-xylene, *m-*xylene, *p*-xylene and any mixture thereof.

4. A process as claimed in claim 1 wherein the Si/Al ratio in said catalyst is between 5 to 20.

5. A process as claimed in claim 2 wherein the temperature of the reaction is in the range of 100 - 200°C.

6. A process as claimed in claim 2 wherein the temperature of the reaction is in the range of 120 - 180°C.

7. A process as claimed in claim 1 wherein the molar ratio of xylene substrate to the alkylating agent in the feed is in the range of from 1:2 to 20:1.

8. A process as claimed in claim 7 wherein the molar ratio of xylene substrate to the alkylating agent is in the range of 1:1 to 10:1.

9. A process as claimed in claim 7 wherein the molar ratio of xylene substrate to the alkylating agent is in the range of 1:2 to 5:1.

10. A process as claimed in claim 1 wherein the weight hourly space velocity (WHSV) of the feed is in the range of 0.5 to 30 h⁻¹.

11. A process as claimed in claim 10 wherein the weight hourly space velocity (WHSV) of the feed is in the range of 1 to 20 h⁻¹.

12. A process as claimed in claim 10 wherein the weight hourly space velocity (WHSV) of the feed is in the range of 2 to 10 h⁻¹.

13. A process as claimed in claim 1 wherein the alkylation reaction is carried out in a fixed bed reactor or a batch reactor.

14. A process as claimed in claim 1 wherein *p*-xylene is alkylated using isopropanol in the presence of zeolite beta catalyst.

15. A process as claimed in claim 1 wherein *m*-xylene and *o*-xylene are alkylated using isopropanol as the alkylating agent in the presence of ultrastable zeolite Y (USY) as catalyst.

16. A process as claimed in claim 1 wherein a mixture of *p*-xylene and isopropyl alcohol in a molar ratio of 4:1 is reacted in a fixed bed reactor in the presence of ultrastable zeolite Y catalyst.

## Patentansprüche

1. Verfahren zur Bereitstellung von Dimethylcumenen in Dampfphase, wobei das Verfahren das Alkylieren eines Substrats, das eines oder mehrere Xylol-Isomeren umfasst, mit einem Alkylans, das aus einer Gruppe ausgewählt wird, die aus Propylen, Isopropanol und n-Propanol besteht, in Gegenwart eines stabilen Säure-ZeolithKatalysators, der aus einem ultrastabilen Zeolith Y mit einem Si/Al-Verhältnis zwischen 5 und 50 und einem Zeolith Beta mit einem Si/Al-Verhältnis zwischen 10 und 120 ausgewählt wurde, und das Trennen der Stoffe umfasst, die in der Dampfphase durch Kondensation bei einer Temperatur in Bereich von 0°C bis 3°C gebildet wurden.

2. Verfahren nach Anspruch 1, wobei das Substrat und das Alkylans mit dem stabilen Säure-Zeolith-Katalysator bei einer Temperatur zwischen 80°C und 250°C und für einen Zeitraum von mindestens einer Stunde in Kontakt gebracht werden.

3. Verfahren nach Anspruch 1, wobei das Substrat aus o-Xylol, m-Xylol, p-Xylol und einer Mischung davon ausgewählt ist.

4. Verfahren nach Anspruch 1, wobei das Si/Al-Verhältnis in dem Katalysator zwischen 5 und 20 beträgt.

5. Verfahren nach Anspruch 2, wobei die Reaktionstemperatur zwischen 100°C und 200°C beträgt.

6. Verfahren nach Anspruch 2, wobei die Reaktionstemperatur zwischen 120°C und 180°C beträgt.

7. Verfahren nach Anspruch 1, wobei das Molverhältnis des Xylol-Substrats zu dem Alkylans in der Zuführung in dem Bereich von 1:2 bis 20:1 liegt.

8. Verfahren nach Anspruch 7, wobei das Molverhältnis des Xylol-Substrats zu dem Alkylans in dem Bereich von 1:1 bis 10:1 liegt.

9. Verfahren nach Anspruch 1, wobei das Molverhältnis des Xylol-Substrats zu dem Alkylans in dem Bereich von 1:2 bis 5:1 liegt.

10. Verfahren nach Anspruch 1, wobei die massenstrombezogene Raumgeschwindigkeit (WHSV) der Zuführung zwischen 0,5 und 30 h⁻¹ beträgt.

11. Verfahren nach Anspruch 1, wobei die auf massenstrombezogene Raumgeschwindigkeit (WHSV) bei der Zuführung zwischen 1 und 20 h⁻¹ beträgt.

12. Verfahren nach Anspruch 1, wobei die massenstrombezogene Raumgeschwindigkeit (WHSV) bei der Zuführung zwischen 2 und 10 h⁻¹ beträgt.

13. Verfahren nach Anspruch 1, wobei die Alkylierungsreaktion in einem Festbettreaktor oder diskontinuierlich betriebenen Reaktor (batch reactor) stattfindet.

14. Verfahren nach Anspruch 1, wobei p-Xylol unter Verwendung von Isopropanol in Gegenwart eines Zeolith-Beta-Katalysators alkyliert wird.

15. Verfahren nach Anspruch 1, wobei m-Xylol und o-Xylol unter Verwendung von Isopropanol als Alkylans in Gegenwart von ultrastabilem Zeolith Y (USY) als Katalysator alkyliert wird.

16. Verfahren nach Anspruch 1, wobei eine Mischung aus p-Xylol und Isopropanol in einem Molverhältnis von 4:1 in einem Festbettreaktor in Gegenwart von ultrastabilem Zeolith-Y-Katalysator eine Reaktion eingeht.

## Revendications

1. Procédé de préparation de diméthylcumènes en phase vapeur, le procédé comprenant l'alkylation d'un substrat comprenant un ou plusieurs isomères de xylène avec un agent alkylant choisi parmi le groupe constitué par le propylène, l'isopropanol et le n-propanol, en présence d'un catalyseur à zéolithe acide sous forme solide choisi parmi la zéolithe Y ultrastable ayant un rapport Si/Al compris dans la fourchette de 5 à 50 et une zéolithe bêta ayant un rapport Si/Al compris dans la fourchette de 10 à 120, et la séparation des produits formés en phase vapeur par condensation à une température comprise dans la fourchette de 0 à 3°C.

2. Procédé tel que revendiqué dans la revendication 1, dans lequel lesdits substrat et agent alkylant sont mis en contact avec ledit catalyseur à zéolithe acide sous forme solide à une température comprise dans la fourchette de 80 à 250°C et pour une durée d'au moins 1 heure.

3. Procédé tel que revendiqué dans la revendication 1, dans lequel le substrat est choisi parmi le *o*-xylène, le *m*-xylène, le *p*-xylène et un quelconque mélange de ceux-ci.

4. Procédé tel que revendiqué dans la revendication 1, dans lequel le rapport Si/Al dans ledit catalyseur est compris entre 5 et 20.

5. Procédé tel que revendiqué dans la revendication 2, dans lequel la température de la réaction est comprise dans la fourchette de 100-200°C.

6. Procédé tel que revendiqué dans la revendication 2, dans lequel la température de la réaction est comprise dans la fourchette de 120-180°C.

7. Procédé tel que revendiqué dans la revendication 1, dans lequel le rapport molaire du substrat de xylène à l'agent alkylant dans l'alimentation est compris entre des valeurs allant de 1:2 à 20:1.

8. Procédé tel que revendiqué dans la revendication 7, dans lequel le rapport molaire du substrat de xylène à l'agent alkylant est compris dans la fourchette allant de 1:1 à 10:1.

9. Procédé tel que revendiqué dans la revendication 7, dans lequel le rapport molaire du substrat de xylène à l'agent alkylant est compris dans la fourchette allant de 1:2 et 5:1.

10. Procédé tel que revendiqué dans la revendication 1, dans lequel la vitesse spatiale horaire en poids (WHSV) de l'alimentation est comprise dans la fourchette allant de 0,5 à 30 h⁻¹.

11. Procédé tel que revendiqué dans la revendication 10, dans lequel la vitesse spatiale horaire en poids (WHSV) de l'alimentation est comprise dans la fourchette allant de 1 à 20 h⁻¹.

12. Procédé tel que revendiqué dans la revendication 10, dans lequel la vitesse spatiale horaire en poids (WHSV) de l'alimentation est comprise dans la fourchette allant de 2 à 10 h⁻¹.

13. Procédé tel que revendiqué dans la revendication 1, dans lequel la réaction d'alkylation est réalisée dans un réacteur à lit fixe ou dans un réacteur en mode discontinu.

14. Procédé tel que revendiqué dans la revendication 1, dans lequel le *p*-xylène est alkylé en utilisant l'isopropanol en présence du catalyseur à zéolithe bêta.

15. Procédé tel que revendiqué dans la revendication 1, dans lequel le *m*-xylène et le *o*-xylène sont alkylés en utilisant l'isopropanol comme agent alkylant en présence de la zéolithe Y ultrastable (USY) comme catalyseur.

16. Procédé tel que revendiqué dans la revendication 1, dans lequel un mélange de *p*-xylène et d'alcool isopropylique dans un rapport molaire de 4:1 est mis à réagir dans un réacteur à lit fixe en présence du catalyseur à zéolithe Y ultrastable.
